# FASCICULE DE BREVET EUROPEEN

(11) **EP 2 956 161 B1**
(45) Date de publication et mention de la délivrance du brevet: **11.11.2020**
(21) Numéro de dépôt: 14708637.5
(22) Date de dépôt: 14.02.2014
(51) Int. Cl.: A61K 38/42, A61P 35/00

(54) **UTILISATION D'HEMOGLOBINE D'ANNELIDES POUR TRAITER LES CANCERS**
VERWENDUNG VON RINGELWURM-HÄMOGLOBIN ZUR BEHANDLUNG VON KREBSERKRANKUNGEN
USE OF ANNELID HEMOGLOBIN FOR THE TREATMENT OF CANCERS

(30) Priorité: 15.02.2013 FR 1351313
(43) Date de publication de la demande: 23.12.2015
(73) Titulaire: Hemarina, 29600 Morlaix (FR)
(72) Inventeur: ZAL, Franck, F-29600 Ploujean-Morlaix (FR)
(74) Mandataire: Lavoix
(86) Numéro de dépôt international: PCT/FR2014/050300
(87) Numéro de publication internationale: WO 2014/125225

(56) Documents cités:
- WO-A1-2010/128159
- WO-A2-2006/096774
- WO-A2-2012/094679
- FR-A1- 2 917 292
- FR-A1- 2 975 869
- JP-A- 2001 348 341
- JP-A- 2009 234 929
- US-A1- 2009 169 591
- US-A1- 2011 295 225
- ROUSSELOT MORGANE ET AL: "Arenicola marina extracellular hemoglobin: a new promising blood substitute", BIOTECHNOLOGY JOURNAL, WILEY-VCH VERLAG, WEINHEIM, DE, vol. 1, no. 3, 1 janvier 2006 (2006-01-01) , pages 333-345, XP002480915, ISSN: 1860-6768, DOI: 10.1002/BIOT.200500049

## Description

La présente invention a trait à l'utilisation d'au moins une hémoglobine extracellulaire de la famille des *Arenicolidae,* pour traiter les cancers, pour diminuer l'hypoxie des cellules cancéreuses, et augmenter de ce fait l'efficacité de traitements de radiothérapie et de chimiothérapie.

Le cancer est une maladie liée à la prolifération anormale et incontrôlable de cellules dites malignes.

Les cancers englobent des tumeurs dites solides, i.e. dans lesquelles les cellules se sont multipliées pour former une masse, et les lymphomes et leucémies où les cellules cancéreuses sont en circulation dans le sang, i.e. ne constituent donc pas de masse solide.

Une diminution de la pression partielle en oxygène (hypoxie tissulaire) a été mise en évidence dans la quasi-totalité des tumeurs solides chez l'homme (Raleigh J, Dewhirst M, Thrall D. Measuring tumor hypoxia. Semin Radiat Oncol 1996 ; 46 : 229-37). Dès les années 1950, l'architecture tumorale a été décrite comme une nécrose hyaline centrale, entourée de tissus viables, à une distance d'environ 100 µm des capillaires (Thomlinson RH, Gray LH. The histological structure of some human lung cancers and possible implications for radiotherapy. Br J Cancer 1955; 9 : 539.). Les tumeurs présentent de larges zones ayant une faible pression partielle en oxygène dans lesquelles les cellules se trouvent en condition d'hypoxie.

Le facteur de transcription hétérodimérique HIF-1 (hypoxia-inducible factor-1) active une grande variété de voies de signalisation permettant à la cellule cancéreuse d'acquérir une réponse adaptée au stress hypoxique. HIF-1 active une série de plus de 50 gènes codant pour des facteurs protéiques impliqués notamment dans la néo-angiogenèse, favorisant la progression tumorale et la dissémination métastatique, le métabolisme du glucose (Glut-1), la survie cellulaire et la chimiorésistance (MDR) (Lauzier MC, Michaud MD, Déry MA, Richard DE. HIF-1 activation during tumor progression : implications and consequences. Bull Cancer 2006 ; 93 : 349-56). La détection de la protéine HIF-1 dans les tumeurs a été corrélée à une diminution de la survie et à une réduction de la sensibilité à la chimiothérapie. Des cellules déficientes pour la protéine HIF-1α montrent en effet une sensibilité accrue à de nombreux agents de chimiothérapie tels que la carboplatine ou l'étoposide (Unruh A, Ressel A, Mohamed HG, Johnson RS, Nadrowitz R, Richter E, et al. The hypoxia-inducible factor-1 alpha is a negative factor for tumor therapy. Oncogene 2003 ; 22 : 3213-20). D'autre part, il a également été montré que l'hypoxie tumorale induit un arrêt du cycle cellulaire en phase G1/S, et augmente la résistance à l'apoptose par l'inactivation du gène suppresseur de tumeurs p53. Il a ainsi été montré dans un modèle de fibrosarcome murin que les cellules hypoxiques sont 2 à 6 fois plus chimiorésistantes que les cellules normoxiques (Teicher BA, Holden SA, Al-Achi A, et al. Classification of antineoplasic treatments by their differential toxicity toward putative oxygenated and hypoxic tumor subpopulations in vivo in the FSaIIC murine fibrosarcoma. Cancer Res 1990 ; 50 : 3339-44).

L'hypoxie est également un facteur majeur de radiorésistance. Les altérations des bases nucléosidiques (cassure simple brin et double brin de l'ADN) peuvent être produites de manière « directe » par dépôt d'énergie sur les brins d'ADN ou, le plus souvent, de manière indirecte par la production de radicaux libres issus de la radiolyse de l'eau. L'oxygène va intervenir au sein de cette cascade radicalaire en permettant la création de radicaux libres à durée de vie plus longue.

L'hypoxie pourrait être considérée comme une cible thérapeutique importante, étant donné sa grande spécificité tumorale (« L'hypoxie tumorale peut-elle devenir un avantage pour la chimiothérapie ? », Tredan et al, Bulletin du Cancer, 2008, vol. 95, n°5, pp. 528-534).

De nouvelles thérapeutiques sont ainsi en cours de développement pour cibler les cellules en condition d'hypoxie. De nombreux agents susceptibles d'inhiber l'expression ou l'activité de HIF-1 sont également en phase préclinique ou clinique.

Afin de pouvoir disposer de thérapies efficaces, il est pertinent d'identifier des thérapies qui inhibent l'hypoxie. En effet, en inhibant ou en diminuant ce mécanisme, les cellules cancéreuses peuvent redevenir sensibles aux traitements de radiothérapie et de chimiothérapie.

II existe donc un besoin pour réduire l'hypoxie de cellules cancéreuses, dans les cas de tumeurs solides.

Les inventeurs ont maintenant découvert que, de façon surprenante, l'hémoglobine extracellulaire d'Annélides permet de diminuer l'hypoxie de cellules cancéreuses, comme cela est démontré en exemple.

La présente invention concerne ainsi l'utilisation d'au moins une hémoglobine extracellulaire de la famille des *Arenicolidae,* pour traiter les cancers, de préférence les tumeurs solides, selon la revendication 1. Le traitement des cancers se fait par diminution de l'hypoxie des cellules cancéreuses ; cela les rend plus sensibles aux traitements de radiothérapie et de chimiothérapie. La présente invention concerne au moins une hémoglobine extracellulaire de la famille des *Arenicolidae,* pour son utilisation pour diminuer l'hypoxie des cellules cancéreuses. La présente description concerne un produit consistant en un agent anti-cancéreux et une hémoglobine extracellulaire de la famille des *Arenicolidae,* comme produit de combinaison pour une utilisation simultanée, séparée ou séquencée, dans le traitement d'un cancer.

De préférence, l'agent anticancéreux est choisi parmi les fludarabine, gemcitabine, capecitabine, methotrexate, taxol, taxotère, mercaptopurine, thioguanine, hydroxyurée, cytarabine, cyclophosphamide, ifosfamide, nitrosourées comme la carmustine et lomustine, complexes de platine comme la cisplatine, carboplatine et l'oxaliplatine, mitomycine, dacarbazine, procarbizine, etoposide, teniposide, campathecines, bleomycine, doxorubicine, idarubicine, daunorubicine, dactinomycine, plicamycine, mitoxantrone, L-asparaginase, epimbicm, 5-fluorouracile, taxanes comme le docetaxel et la paclitaxel, leucovorine, levamisole, irinotecan (CPT-11), SN-38, estramustine, moutarde d'azote, BCNU, vinblastine, vincristine et vinorelbine, les anticorps monoclonaux contre le récepteur EGF ou VEGF, comme le bevacizumab, cetuximab et panitumumab, imatimb mesylate, hexamethyhnelamine, topotecan, genisteine, erbstatine, lavendustine et aussi le bortezomib (ou PS341, vendu par Millenium Pharmaceuticals sous le nom Velcade).

L'hémoglobine extracellulaire d'Annélides est présente chez les trois classes d'Annélides : les Polychètes, les Oligochètes et les Achètes. On parle d'hémoglobine extracellulaire car elle est naturellement non contenue dans une cellule, et peut donc circuler librement dans le système sanguin sans modification chimique pour la stabiliser ou la rendre fonctionnelle.

L'hémoglobine extracellulaire d'Annélides est un biopolymère géant de poids moléculaire compris entre 2000 et 4000 kDa, constitué d'environ 200 chaînes polypeptidiques comprises entre 4 et 12 types différents que l'on regroupe généralement en deux catégories.

La première catégorie, comptant 144 à 192 éléments, regroupe les chaînes polypeptidiques dites "fonctionnelles" qui portent un site actif de type hème, et sont capables de lier réversiblement l'oxygène ; ce sont des chaînes de type globine dont les masses sont comprises entre 15 et 18 kDa et qui sont très similaires aux chaînes de type α et β de vertébrés.

La deuxième catégorie, comptant 36 à 42 éléments, regroupe les chaînes polypeptidiques dites de "structure" ou « linkers » possédant peu ou pas de site actif mais permettant l'assemblage des sous-unités appelées douzièmes ou protomères.

Chaque molécule d'hémoglobine est constituée de deux hexagones superposés que l'on a nommés bicouche hexagonale (hexagonal bilayer) et chaque hexagone est lui-même formé par l'assemblage de six sous-unités (dodécamère ou protomère) en forme de goutte d'eau. La molécule native est formée de douze de ces sous-unités (dodécamère ou protomère). Chaque sous-unité a une masse moléculaire d'environ 250 kDa, et constitue l'unité fonctionnelle de la molécule native.

L'hémoglobine extracellulaire est de la famille des *Arenicolidae* . Encore plus préférentiellement, l'hémoglobine extracellulaire est l'hémoglobine extracellulaire *d'Arenicola sp,* plus préférentiellement l'hémoglobine extracellulaire *d'Arenicola marina.*

Selon la description, le protomère de globine de l'hémoglobine extracellulaire de la famille des *Arenicolidae* constitue l'unité fonctionnelle de l'hémoglobine native, comme indiqué ci-dessus. Enfin, la chaîne de globine de l'hémoglobine extracellulaire des *Arenicolidae* peut notamment être choisie parmi les chaînes de globine de type Ax et/ou Bx d'hémoglobine extracellulaire des *Arenicolidae.*

L'hémoglobine extracellulaire de la famille des *Arenicolidae,* ses protomères de globine et/ou ses globines ne nécessitent pas de cofacteur pour fonctionner, contrairement à l'hémoglobine de mammifère, notamment humaine. Enfin, l'hémoglobine extracellulaire de la famille des *Arenicolidae,* ses protomères de globine et/ou ses globines ne possédant pas de typage sanguin, ils permettent d'éviter tout problème de réaction immunologique.

L'hémoglobine extracellulaire de la famille des *Arenicolidae,* ses protomères de globine et/ou ses globines peuvent être natifs ou recombinants.

Selon l'invention, l'hémoglobine extracellulaire de la famille des *Arenicolidae* est présente dans une composition comprenant une solution tampon.

Ladite solution tampon créée un environnement salin adéquat pour l'hémoglobine, ses protomères et ses globines, et permet ainsi le maintien de la structure quaternaire, et donc de la fonctionnalité de cette molécule. Grâce à la solution tampon, l'hémoglobine, ses protomères et ses globines sont capables d'assurer leur fonction d'oxygénation.

La solution tampon selon l'invention est une solution aqueuse comprenant des sels, de préférence des ions chlorure, sodium, calcium, magnésium et potassium, et confère à la composition selon l'invention un pH compris entre 6.5 et 7.6 ; sa formulation est similaire à celle d'un liquide physiologiquement injectable. Dans ces conditions, l'hémoglobine extracellulaire d'Annélides, ses protomères de globine et ses globines restent fonctionnels.

Dans la présente description, le pH s'entend à température ambiante (25°C), sauf mention contraire.

De préférence, la solution tampon est une solution aqueuse comprenant du chlorure de sodium, du chlorure de calcium, du chlorure de magnésium, du chlorure de potassium, ainsi que du sodium gluconate et du sodium acétate, et a un pH compris entre 6.5 et 7.6, de préférence égal à 7,1 ± 0,5, de préférence d'environ 7.35. Plus préférentiellement, la solution tampon est une solution aqueuse comprenant 90 mM de NaCl, 23 Mm de Na-gluconate, 2,5 mM de CaCl₂, 27 mM de Na-acétate, 1,5 mM de MgCl₂, 5 mM de KCl, et a un pH de 7,1 ± 0,5, pouvant contenir entre 0 et 100 mM d'antioxydant de type acide ascorbique et/ou glutathion réduit.

De préférence, la composition est administrée au sujet par voie parentérale, de préférence par injection ou perfusion.

De préférence, la composition comprenant l'hémoglobine, ses protomères ou ses globines, et la solution tampon est administrée en tant que telle. En effet, dans ce cas, l'hémoglobine, ses protomères ou ses globines, est présente dans une composition comprenant une solution tampon, de préférence une solution aqueuse comprenant des sels, et conférant à la composition un pH compris entre 6.5 et 7.6. De préférence, la composition contient uniquement l'hémoglobine, ses protomères ou ses globines, et une solution tampon consistant en une solution aqueuse comprenant des sels, et conférant à la composition un pH compris entre 6.5 et 7.6. La galénique d'administration est donc assez simple et efficace.

De préférence, les cancers sont choisis parmi les carcinomes, les sarcomes, les mélanomes, les cancers du sein, du côlon, de l'ovaire, de la prostate, de l'utérus, du foie, du poumon et de la thyroïde.

L'invention est décrite plus en détail dans les exemples suivants. Ces exemples sont fournis à des fins d'illustration uniquement, et ne sont pas limitatifs.

Les figures sont illustrées par les légendes suivantes :

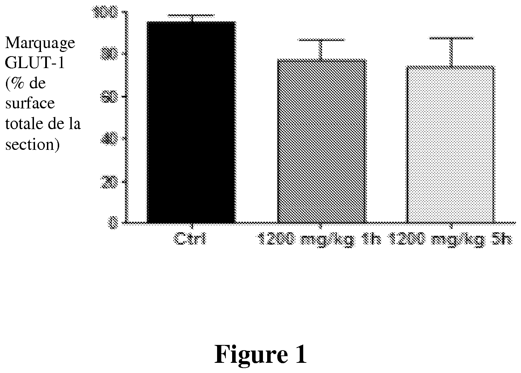

Figure 1 : Quantification du marquage anti-GLUT-1 sur des sections de tissu tumoral dans le groupe contrôle (Ctrl), et 1h et 5h après injection iv de 1200 mg/kg M101. Le signal positif observé sur les tranches de paraffine a été exprimé en fraction d'aire de cellules tumorales marquées comparée à la surface totale de la tumeur. Les valeurs correspondent à la moyenne de 3 échantillons (moyenne +/- SD).

### Exemples

### Matériel :

### Arenicola marina (M101):

Deux lots à la concentration de 100 mg/mL et 178 mg/mL ont été utilisés pour cette étude. M101 est conservé à -80°C et décongelé à 4°C pour les études.

La dilution de M101 à la concentration étudiée a été effectuée avec le tampon de stabilisation M101 : 4 mM KCl, 145 mM NaCl, 0,2 mM MgCl2, 10 mM Hepes, 0,1 M NaOH ; pH7.

### Modèle tumoral in vivo, traitement par M101 et analyses immunohistochimiques des tumeurs. :

Des cellules HT29 d'adénocarcinome colique humain (ATCC, HTB-38) ont été cultivées dans du milieu DMEM supplémenté avec 20% de FBS, 1% de L-glutamine et 1% d'antibiotiques (pénicilline et streptomycine) à 37°C dans une atmosphère saturée en eau, à 5% CO2/ 95% d'air.

Les cellules HT29 amplifiées *in vitro* (3.10⁶ cellules) ont été injectées en sous-cutané au niveau du flanc droit de souris Nude âgées de 6-8 semaines, préalablement irradiées à la dose de 5 Gy. Lorsque le diamètre des tumeurs a atteint ∼ 5 mm, les souris ont été injectées par voie intraveineuse avec M101 à 3 concentrations différentes (60, 600 et 1200 mg/kg). La capacité de M101 à réduire l'hypoxie dans les tumeurs a été suivie au cours du temps, par quantification de l'expression de GLUT-1 par immunohistochimie (anticorps anti-GLUT1 et détection par streptavidine-biotine). Pour ce faire, les souris ont été euthanasiées à différents temps après le traitement par M101 et des tumeurs HT29 prélevées à 5 min, 15 min, puis à 1, 2, 3, 4, 5, 6 et 24 h. GLUT1 a été évaluée comme marqueur intrinsèque de l'hypoxie dans les tissus (Gbadamosi JK, Hunter AC, Moghimi SM (2002) PEGylation of microspheres generates a heterogeneous population of particles with differential surface characteristics and biological performance. FEBS Lett 532:338-344; Pionetti JM, Pouyet J (1980) Molecular architecture of annelid erythrocruorins. Extracellular hemoglobin of Arenicola marina (Polychaeta). Eur J Biochem 105:131-138.)

### Résultats

### Cinétique de l'oxygénation du tissu tumoral.

La capacité d'oxygénation de M101 a été déterminée *in vivo* par évaluation de la réduction du niveau d'hypoxie des tumeurs sous-cutanées HT29 par quantification immunohistologique du marqueur GLUT-1 (tableau 1 ci-dessous).

**Tableau 1**

| | **Dose injectée (mg/kg)** | | |
|---|---|---|---|
| | 60 | 600 | 1200 |
| Contrôle | +++ | +++ | +++ |
| 5 min | +++ | ++/- | +/- |
| 15 min | +++ | ++/- | +/- |
| 1 h | +++ | +/- | + |
| 2 h | +++ | + | + |
| 3 h | +++ | +/- | + |
| 4 h | +++ | +/- | + |
| 5 h | +++ | +/- | +/- |
| 6 h | ++/- | + | + |
| 24 h | +++ | +/- | +/- |

| | | | |
|---|---|---|---|
| *Le niveau d'hypoxie tissulaire est évalué par quantification du marquage Glut-1 au cours du temps après injection intraveineuse de M101.* *Le niveau d'hypoxie tissulaire est déterminé selon l'échelle suivante:* "+++", *marquage Glut-1 très intense, très forte hypoxie tissulaire* "*++*/*-*", *marquage Glut-1 intense, hypoxie tissulaire forte* "*+*": "*+*/*-* ", *marquage Glut-1 intermédiaire, hypoxie tissulaire réduite* "*+*/*-*": *marquage Glut-1 faible, hypoxie tissulaire faible* | | | |

Après numérisation des lames histologiques, un logiciel dédié a permis de mesurer le niveau d'hypoxie pour chaque condition. L'examen d'échantillons de tissus tumoraux après traitement (injection de M101 à 600 mg/kg et 1200 mg/kg) a montré que les zones d'hypoxie ont été réduites comparées aux contrôles, et sont remplacées par un tissu fibreux qui tend à se dissocier et infiltrer la tumeur. L'administration iv de M101 à la dose de 1200 mg/kg réduit le niveau de l'hypoxie tumorale de 20% en moyenne au bout d'1 heure (atteignant jusqu'à 40%) et de 23% après 5 heures (voir Figure 1).

Ces données suggèrent que M101 est capable de diffuser dans le tissu tumoral et de réduire l'intensité de la coloration de GLUT-1, mettant ainsi en évidence son effet sur l'oxygénation tumorale.

## Revendications

1. Hémoglobine extracellulaire de la famille des *Arenicolidae,* pour son utilisation pour diminuer l'hypoxie des cellules cancéreuses et traiter un cancer chez un sujet traité par radiothérapie, l'hémoglobine étant présente dans une composition comprenant une solution tampon.

2. Hémoglobine pour son utilisation selon la revendication 1, **caractérisée en ce que** l'hémoglobine extracellulaire de la famille des *Arenicolidae* est l'hémoglobine extracellulaire d'*Arenicola sp.*

3. Hémoglobine pour son utilisation selon l'une des revendications 1 ou 2 , **caractérisée en ce que** l'hémoglobine extracellulaire de la famille des *Arenicolidae* est l'hémoglobine extracellulaire d'*Arenicola marina.*

4. Hémoglobine pour son utilisation selon l'une des revendications 1 à 3, **caractérisée en ce que** les cancers sont des tumeurs solides.

5. Hémoglobine pour son utilisation selon l'une des revendications 1 à 4, **caractérisée en ce que** les cancers sont choisis parmi les carcinomes, les sarcomes, les mélanomes, les cancers du sein, du côlon, de l'ovaire, de la prostate, de l'utérus, du foie, du poumon et de la thyroïde.

6. Hémoglobine pour son utilisation selon l'une des revendications 1 à 5, **caractérisée en ce que** l'hémoglobine est présente dans une composition comprenant une solution aqueuse comprenant des sels, et conférant à la composition un pH compris entre 6.5 et 7.6.

## Patentansprüche

1. Extrazelluläres Hämoglobin der Familie *Arenicolidae* für seine Verwendung zum Verringern der Hypoxie von Krebszellen und Behandeln einer Krebserkrankung bei einer durch Strahlentherapie behandelten Versuchsperson, wobei das Hämoglobin in einer Zusammensetzung vorhanden ist, welche eine Pufferlösung aufweist.

2. Hämoglobin für seine Verwendung gemäß Anspruch 1, **dadurch gekennzeichnet, dass** das extrazelluläre Hämoglobin der Familie *Arenicolidae* das extrazelluläre Hämoglobin der *Arenicola sp* ist.

3. Hämoglobin für seine Verwendung gemäß einem der Ansprüche 1 und 2, **dadurch gekennzeichnet, dass** das extrazelluläre Hämoglobin der Familie *Arenicolidae* das extrazelluläre Hämoglobin der *Arenicola marina* ist.

4. Hämoglobin für seine Verwendung gemäß einem der Ansprüche 1 bis 3, **dadurch gekennzeichnet, dass** die Krebserkrankungen solide Tumore sind.

5. Hämoglobin für seine Verwendung gemäß einem der Ansprüche 1 bis 4, **dadurch gekennzeichnet, dass** die Krebserkrankungen ausgewählt sind aus den Karzinomen, den Sarkomen, den Melanomen, den Krebserkrankungen der Brust, des Darms, der Eierstöcke, der Prostata, des Uterus, der Leber, der Lunge und der Schilddrüse.

6. Hämoglobin für seine Verwendung gemäß einem der Ansprüche 1 bis 5, **dadurch gekennzeichnet, dass** das Hämoglobin in einer Zusammensetzung vorhanden ist, welche eine wässrige Lösung aufweist, die Salze aufweist und der Zusammensetzung einen pH zwischen 6,5 und 7,6 verleiht.

## Claims

1. Extracellular hemoglobin of the family *Arenicolidae,* for use for reducing hypoxia in cancer cells and treating cancer in a subject treated by radiotherapy, wherein the hemoglobin is present in a composition comprising a buffer solution.

2. Hemoglobin as claimed in claim 1, **characterized in that** the extracellular hemoglobin of the family *Arenicolidae* is the extracellular hemoglobin of *Arenicola sp.*

3. Hemoglobin as claimed in claim 1 or 2, **characterized in that** the extracellular hemoglobin of the family *Arenicolidae* is the extracellular hemoglobin of *Arenicola marina.*

4. Hemoglobin as claimed in any one of claims 1 to 3, **characterized in that** the cancers are solid tumors.

5. Hemoglobin as claimed in any one of claims 1 to 4, **characterized in that** the cancers are chosen from carcinomas, sarcomas, melanomas, breast cancer, colon cancer, ovarian cancer, prostate cancer, uterine cancer, liver cancer, lung cancer and thyroid cancer.

6. Hemoglobin as claimed in any one of claims 1 to 5, **characterized in that** the hemoglobin is present in a composition comprising an aqueous solution comprising salts and which gives the composition a pH of between 6.5 and 7.6.
